(19) Europäisches Patentamt
European Patent Office
Office européen des brevets

(11) Publication number: 0 402 472 A1

(12) EUROPEAN PATENT APPLICATION
published in accordance with Art. 158(3) EPC

(21) Application number: 89909851.1

(22) Date of filing: 05.09.89

(86) International application number: PCT/JP89/00911

(87) International publication number: WO 90/02734 (22.03.90 90/07)

(51) Int. Cl.5: C07D 207/34, C07D 209/42

(30) Priority: 06.09.88 JP 221405/88

(43) Date of publication of application: 19.12.90 Bulletin 90/51

(84) Designated Contracting States: AT BE CH DE FR GB IT LI LU NL SE

(71) Applicant: NIPPON SODA CO., LTD.
2-1, Ohtemachi 2-chome
Chiyoda-ku, Tokyo 100(JP)

(72) Inventor: YAGIHARA, Tomio Mizushima Plant Nippon Soda
2767-12, Aza Niihama Shionasu, Kojima
Kurashiki-shi Okayama 711(JP)
Inventor: MATSUI, Nobuo Odawara Research Center Nippon Soda
345, Aza Yanagimachi Takada
Odawara-shi Kanagawa 250-02(JP)
Inventor: HAMAMOTO, Isami Odawara Research Center
Nippon Soda 345, Aza Yanagimachi Takada
Odawara-shi Kanagawa 250-02(JP)

(74) Representative: de Bruijn, Leendert C. et al
Nederlandsch Octrooibureau
Scheveningseweg 82 P.O. Box 29720
NL-2502 LS 's-Gravenhage(NL)

(54) PYRROLE DERIVATIVES AND PROCESS FOR THEIR PREPARATION.

(57)

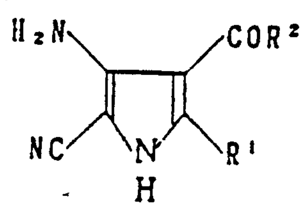

(I)

The invention relates to compounds represented by general formula (I), [wherein $R^1$ represents a hydrogen

EP 0 402 472 A1

atom, a lower alkyl group or an aryl group, $R^2$ represents a lower alkyl group, a group represented by -Xr (wherein X represents an oxygen atom or $Nr^1$, r represents a lower alkyl group or an aryl group, and $r^1$ represents a hydrogen atom or a lower alkyl group) or an aryl group, provided that $R^1$ and $-COR^2$ may be combined together to form a ring] and a process for their preparation. The compounds are useful as starting materials for agrochemicals and medicines, porphyrins, dyes, etc.

TITLE MODIFIED
see front page

Specification

Pyrrole derivative and its preparation method

〔Technical Field〕

This invention relates to new pyrrole derivatives useful as starting materials for such products as agricultural chemicals, pharmaceuticals, porphyrin and dyes.

〔Background Art〕

Pyrrole derivatives are important chemical substances as physiologically active substances or functional substances. Known synthetic methods for pyrrole derivatives include ① reaction between diketones and amine, ② reaction between isonitrile and α, β-unsaturated ketone, ③ reaction between acyloin and β-aminocrotonic acid, and ④ reaction between haloketone and β-aminocrotonic acid. Synthetic methods are scarcely known for pyrrole derivatives having a cyano group and an amino group at the same time as substituents of pyrrole ring, like the compounds of this invention.

A pyrrole compound having an amino group and a cyano group at the same time as substituents is an important intermediate with capabilities for synthesis of pyrimidine derivatives important in physiological activities by using the groups.

The inventors of this invention studied synthetic methods for pyrrole derivatives, quite different from conventional methods.

〔Disclosure of Invention〕

The inventors earnestly studied preparation methods for pyrrole derivatives having a cyano group and an amino group at the same time as substituents of the pyrrole ring, resulting in finding that the intended compound, 3-amino-2-cyano pyrrole derivative is obtained from a reaction between diamino acrylonitrile

derivative and active methylene compound via Schiff base, with good yield.

That is, this invention is

(1) a compound represented by general formula

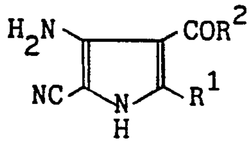

(I)

[where, $R^1$ is a hydrogen atom, a lower alkyl group or an aryl group, and $R^2$ is a lower alkyl group or a group represented by a formula, -Xr (where X is an oxygen atom or $Nr^1$,r is a lower alkyl group or an aryl group, and $r^1$ is a hydrogen atom or a lower alkyl group) or an aryl group; however $R^1$ and $-COR^2$ can form a ring together] , and

(2) a method for the preparation of compound represented by general formula

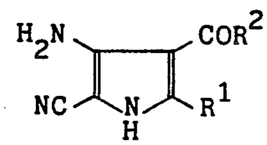

(I)

(where $R^1$ and $R^2$ are as defined above) which comprises that a Schiff base obtained from a compound represented by general formula

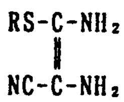

(II)

(where R is an alkyl group, an aryl group or an aralkyl group) and a compound represented by general formula

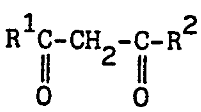

(III)

(where, $R^1$ and $R^2$ are as defined above) is reacted to form a ring in the presence of base.

[Best Mode for Carrying Out of the Invention]

In the preparation method of this invention, a Schiff base is prepared under usual conditions for preparing a Schiff base, that is, a dehydration reaction is carried out in organic solvents, in the presence of catalyst such

as p-toluene sulfonic acid or oxalic acid if necessary, or using a dehydrating agent such as phosphorus pentaoxide.

Solvents used are organic solvents inactive to the reaction, including alcohols; ethers; acetonitrile; esters such as ethyl acetate; amides such as DMF; and hydrocarbons such as benzene and xylene.

After the reaction is completed, the product is isolated by distilling the solvent or by filtrating, or is not isolated keeping it as the reaction solvent or suspension, and is used for the next ring formation reaction.

The ring formation reaction is carried out in organic solvents in the presence of base, usually at room temperature, or by heating if necessary.

Solvents used can be the same solvents as those used for preparing Schiff bases. If hydrocarbons are used, it is preferable to use a mixture solvent with alcohols.

A base such as alcoholate, sodium hydroxide, potassium hydroxide, sodium hydride or potassium hydride is appropriately selected depending on reaction solvents used.

After the reaction is completed, usual after-treatment gives the intended compound.

The compound structure is determined by such means as IR, NMR and MASS.

A starting material compound represented by general formula [II] is, for example, prepared according to the following reaction equation.

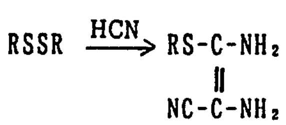


[II]

This invention is further described in detail by reference to the following examples.

Example 1

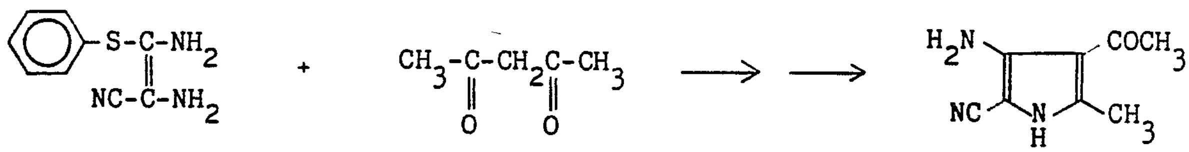

(a) 19.1 g of 2,3-diamino-3-phenylthioacrylonitrile and 10g of acetyl acetone were dissolved in 200ml of ethyl acetate to stir for a day and night.

The deposited crystal was filtrated to give 18.0g of Schiff base.

13.6g of the obtained Schiff base was dissolved in 100ml of methanol, to which 9.6g of 28% sodium methylate was added. The resulting solution was heated under reflux for 2 hours.

After the solution was cooled to room temperature, the deposited crystal was filtrated to give 5.6g of crystal.

Methanol was distilled from the filtrate to concentrate to about half in volume, and the residue was filtrated to give additional 1.2g of crystal.

The crystal combined with the previous one was washed with methanol and recrystallized from methanol to give 6.1g of 3-acetyl-4-amino-5-cyano-2-methylpyrrole (Compound No. 4). m.p. 328 - 330℃ (decomposed)

(b) 2.7g of the Schiff base obtained in (a) was dissolved in 20ml of DMF, to which 0.4g of 60% sodium hydride was added at room temperature. The resulting mixture was stirred at the same temperature for 2 hours. The reaction mixture was poured into 100ml of ice water, and extracted with ethyl acetate. Ethyl acetate was distilled and the residue was recrystallized from methanol to give 0.9g of the intended product. Its IR spectrum was completely the same as that of product obtained in (a).

Example 2

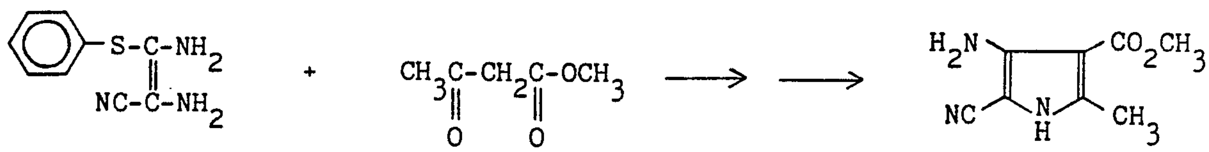

A dehydration reaction was carried out in a benzene solution of 5.7g of 2,3-diamino-3-phenylthioacrylonitrile and 3.5g of methyl acetoacetate in the presence of catalytic amount of oxalic acid, using a Dean-Stark tube. The reaction solution was cooled to room temperature, and the deposited crystal was filtrated to give 5.4g of Schiff base.

5.4g of the obtained Schiff base was dissolved in 50ml of methanol, to which 3.6g of 28% sodium methylate was added. The resulting solution was heated under reflux with stirring for an hour. After the reaction was completed, the solution was cooled. The deposited crystal was filtrated and recrystallized from methanol to give 1.7g of 3-amino-2-cyano-4-methoxycarbonyl-5-methylpyrrole (Compound No. 1). m.p. 229 - 231℃

Example 3

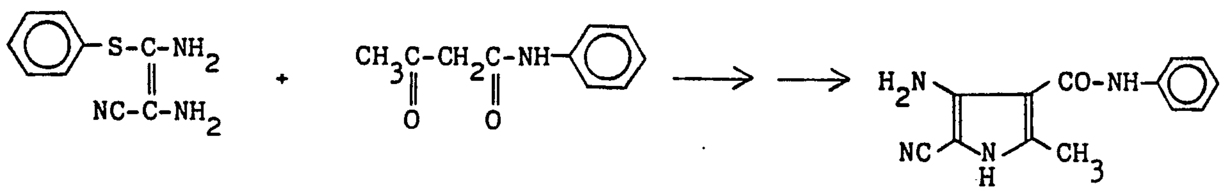

Example 2 was repeated except using 1.9g of 2,3-diamino-3-phenylthioacrylonitrile and 1.8g of acetoacetate anilide. One gram of Schiff base was obtained. 0.9g of the obtained Schiff base was dissolved in 20ml of methanol, to which 0.46g of 28% sodium methylate was added. The resulting mixture was stirred for a day and night.

The reaction mixture was poured into 100ml of water and extracted with ethyl acetate twice.

Ethyl acetate was distilled and the residue was recrystallized from ethanol to give 0.37g of 3-amino-2-cyano-4-phenylcarbamoyl-5-methylpyrrole (Compound No. 3). m.p. 196 - 197.5℃

Typical examples of the compounds of this invention, prepared in a similar way, are shown in Table 1, including the above examples.

Table 1

| Compound No. | Structural formula 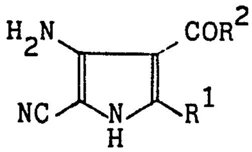 R¹ | R² | Physical constant [ ] ℃ melting point |
|---|---|---|---|
| 1 | $-CH_3$ | $-OCH_3$ | [229-231 ] |
| 2 | " | $-OC_2H_5$ | [227-229 ] |
| 3 | " | 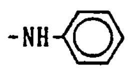 | [196-197.5 ] |
| 4 | " | $-CH_3$ | [328-330 ] decomposed |
| 5 | " | 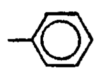 | [303.5-306 ] decomposed |
| 6 | R¹ -COR² | 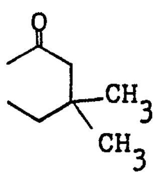 | 300℃ or above |
| 7 | $-CH_3$ | 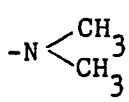 | [199-200 ] |

[Industrial Applicability]

The compounds of this invention, as described above, have a cyano group and an amino group at the same time, thereby being possible to derive to various compounds.

For example, pyrrolopyrimidine derivatives having a skeleton similar to that of purine constituting of bioconstituents of nuclear acids and coenzymes can be easily synthesized. Therefore the compounds of this invention are expected to be applied to the medical and pharmaceutical fields.

## Claims

(1) A compound represented by general formula

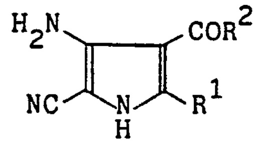

[where, $R^1$ is a hydrogen atom, a lower alkyl group or an aryl group, and $R^2$ is a lower alkyl group or a group represented by a formula -Xr (where X is an oxygen atom or $Nr^1$, r is a lower alkyl group or an aryl group, and $r^1$ is a hydrogen atom or a lower alkyl group) or an aryl group; however $R^1$ and $-COR^2$ can form a ring together].

(2) A method for the preparation of compound represented by general formula

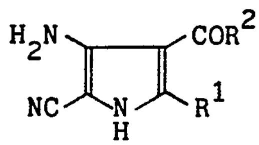

(where $R^1$ and $R^2$ are as defined above) which comprises that a Schiff base obtained from a compound represented by general formula

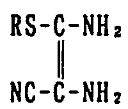

(where R is an alkyl group, an aryl group or an aralkyl group) and a compound represented by general formula

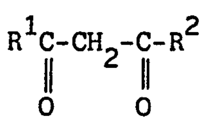

(where, $R^1$ and $R^2$ are as defined above) is reacted to form a ring in the presence of base.

8990985/1.1 0070

# INTERNATIONAL SEARCH REPORT

International Application No PCT/JP89/00911

**I. CLASSIFICATION OF SUBJECT MATTER** (if several classification symbols apply, indicate all)

According to International Patent Classification (IPC) or to both National Classification and IPC

Int. Cl[4] C07D207/34, 209/42

**II. FIELDS SEARCHED**

Minimum Documentation Searched

| Classification System | Classification Symbols |
|---|---|
| IPC | C07D207/34, 209/42 |

Documentation Searched other than Minimum Documentation to the Extent that such Documents are Included in the Fields Searched

**III. DOCUMENTS CONSIDERED TO BE RELEVANT**

| Category * | Citation of Document, with indication, where appropriate, of the relevant passages | Relevant to Claim No. |
|---|---|---|
| A | Chemical Abstracts. 106(7):50164j (Arch. Pharm. (Weinheim, Ger.), 319(9), (1986), Seitz, Gunther, et al [Reactivity of electron-rich CN multiple bonds toward acceptor-substituted 1, 2, 4-triazines], P.798 - 807) | 1 - 2 |

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

**IV. CERTIFICATION**

| Date of the Actual Completion of the International Search | Date of Mailing of this International Search Report |
|---|---|
| October 3, 1989 (03. 10. 89) | October 16, 1989 (16. 10. 89) |
| International Searching Authority | Signature of Authorized Officer |
| Japanese Patent Office | |